(19) Europäisches Patentamt
European Patent Office
Office européen des brevets

(11) Numéro de publication: **0 181 475**
**B1**

(12) # FASCICULE DE BREVET EUROPÉEN

(45) Date de publication du fascicule du brevet: **08.06.88**

(51) Int. Cl.⁴: **C 07 D 303/32,** A 61 K 7/46

(21) Numéro de dépôt: **85112337.2**

(22) Date de dépôt: **28.09.85**

(54) **Cétone aliphatique non saturée et son utilisation à titre d'ingrédient parfumant.**

(30) Priorité: **15.10.84 CH 4926/84**

(43) Date de publication de la demande: **21.05.86 Bulletin 86/21**

(45) Mention de la délivrance du brevet: **08.06.88 Bulletin 88/23**

(84) Etats contractants désignés: **CH DE FR GB LI NL**

(56) Documents cité:
**CH-A-536 834**
**US-A-3 931 326**

(73) Titulaire: **FIRMENICH SA, 1, route des Jeunes, CH-1211 Genève 8 (CH)**

(72) Inventeur: **Schulte- Elte, Karl- Heinrich, Dr., 44, chemin de Carabot, CH- 1213 Onex (CH)**
Inventeur: **Kastner, Dietrich, Dr., Chemin Châtel Dessus, CH- 1261 Givrins (CH)**

(74) Mandataire: **Salvadori, Giuseppe, Dr., c/o Firmenich S.A. Case Postale 239, CH- 1211 Genève 8 (CH)**

**EP 0 181 475 B1**

Il est rappelé que: Dans un délai de neuf mois à compter de la date de publication de la mention de la délivrance du brevet europeen toute personne peut faire opposition au brevet européen délivré, auprés de l'Office européen des brevets. L'opposition doit être formée par écrit et motivée. Elle n'est réputée formée qu'aprés paiement de la taxe d'opposition (Art 99(1) Convention sur le brevet européen).

**Description**

La présente invention a trait au domaine de la parfumerie; en particulier, elle concerne une cétone aliphatique non saturée de formule

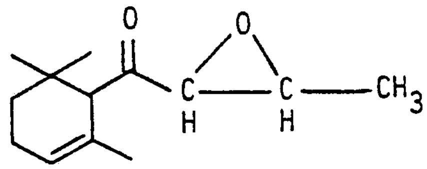

et son utilisation à titre d'ingrédient parfumant.

L'art antérieur fait état de nombreux composés dont la structure est apparentée à celle des damascones ou de la β-damascénone. Depuis leur découverte en effet [voir brevet suisse n° 520 479], l'intérêt porté à ces composés n'a cessé de grandir et fort nombreuses en sont les applications industrielles tant dans le domaine de la parfumerie que dans celui des arômes.

Parmi les composés cités connus figurent les époxydes de formule

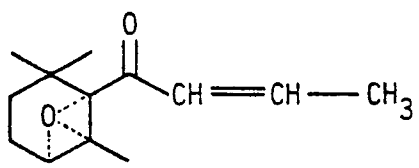

[voir brevet suisse n° 536 834], lesquels composés sont utilisés à titre de produits intermédiaires dans un procédé particulier de préparation de la β-damascénone. Le brevet US n° 3 931 326 mentionne également leurs propriétés organoleptiques [voir notamment l'exemple 40, colonne 53] sans toutefois en indiquer les caractères spécifiques.

Nous avons maintenant découvert que l'époxy-cétone de formule (I) possède des propriétés olfactives utiles et peut, de ce fait, trouver un emploi avantageux dans des applications variées en parfumerie alcoolique et, surtout, en parfumerie technique. Nous avons découvert que ledit composé de l'invention peut se prêter tout spécialement au parfumage d'articles divers tels les savons et les détergents liquides ou solides cationiques, anioniques, non ioniques ou zwitterioniques, ou les adoucissants textiles. Dans des milieux typiquement agressifs, tels ceux représentés par certains types de détergents, le composé de l'invention s'est montré parfaitement stable; il possède une bonne substantivité sur les textiles tant à fibre naturelle qu'artificielle et sa performance olfactive est homogène dans le temps. D'autre part, il est apparu que ce composé est inoffensif pour l'homme et l'environnement et son utilisation peut donc être envisagée dans une gamme de concentrations élevées et d'utilisations variées.

Au point de vue de ses caractéristiques odorantes, le composé de l'invention se différencie des composés apparentés de l'art antérieur par le fait de posséder une note fleurie, fruitée dans laquelle cependant la note de pomme typique de l'α-damascone est également présente, mais de façon amoindrie. Il ne possède en outre pas le caractère légèrement camphré propre à certaines qualités de l'α-damascone et développe, au contraire, une légère note terreuse, verte et naturelle. Vis-à-vis de l'isomère décrit dans l'art antérieur de formule

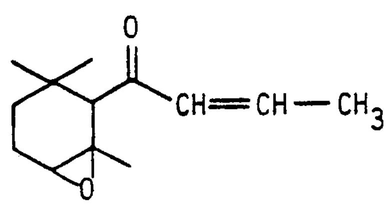

la cétone de l'invention possède une odeur tout à fait différente et plus noble. L'isomère cité développe en effet un caractère vineux, terpénique, camphré, voire moisi. Leurs champs d'application sont donc bien distincts.

Cette observation confirme, si besoin était, le caractère d'incertitude qui entoure toute théorie relative à la perception olfactive. L'expérience montre en effet que toute modification de l'arrangement atomique dans une molécule donnée produit des modifications parfois sensibles dans ses propriétés odorantes, sans qu'on puisse en définir les raisons véritables.

La cétone de l'invention peut être utilisée dans une gamme de concentrations assez large. Sa puissance est à considérer comme moyenne et c'est ainsi que des proportions de l'ordre de 5 à 10, voire 20 %, peuvent parfois être nécessaires. L'homme de l'art sait par expérience que de telles valeurs peuvent varier en fonction de la nature des produits que l'on désire parfumer ou de celle des coingrédients présents dans une composition donnée, ainsi que des effets particuliers qu'on désire atteindre. Lors du parfumage de savons ou détergents

par exemple, des concentrations de l'ordre de 1 ou 2 % peuvent être suffisantes.

Le composé de l'invention peut être utilisé en tant qu'ingrédient unique, mais de préférence, il est employé en mélange avec des coingrédients parfumants usuels (à titre d'exemple on peut citer à cet effet les composés naturels ou synthétiques mentionnés dans la demande de brevet européen publiée sous le n° 0 096 243).

L'époxy-cétone de l'invention est une entité chimique nouvelle. Elle est obtenue conformément à l'invention par un procédé qui consiste en l'époxydation de l'α-damascone au moyen d'un agent d'époxydation en milieu basique. C'est ainsi qu'on utilise comme agent d'époxydation préférentiel l'eau oxygénée et que l'on opère dans une solution aqueuse ou aqueuse-alcoolique d'hydroxyde de sodium ou de potassium. L'époxydation de l'α-damascone peut également s'effectuer au moyen d'un perborate d'un métal alcalin en milieu aqueux basique.

Il est apparent que l'époxy-cétone de l'invention, définie à l'aide de la formule (I), est caractérisée par plusieurs centres d'asymétrie dans sa molécule. Il demeure entendu que la formule donnée sert à définir tout composé épimère ou énantiomère possible. Pour des raisons d'ordre pratique et économique, on utilisera, pour tout emploi conforme à l'invention, un mélange tel que directement obtenu par le procédé d'époxydation décrit plus haut.

L'invention est illustrée plus en détail dans les exemples suivants dans lesquels les températures sont indiquées en degrés centigrades et les abréviations ont le sens usuel dans l'art.

**Exemple 1**

Préparation de 2,3-époxy-1-(2,6 6-triméthyl-2-cyclohexène-1 -yl)-1-butanone

A une solution de 3,9 g d'α-damascone, 4,6 g d'eau oxygénée à 30 % et 200 ml de méthanol on a ajouté 1,2 ml de NaOH 6N tout en refroidissant le réacteur à l'aide d'un bain extérieur, puis le mélange a été maintenu à température ambiante pendant 48 h et dilué ensuite avec 200 ml d'eau, puis extrait plusieurs fois à l'éther.

Après séchage et évaporation on a obtenu 4,1 g d'un résidu qui par distillation dans un four à bulles a fourni 3,8 g (rend. 90 %) de l'époxy-cétone désirée sous forme d'un mélange de diastéréoisomères.

$n_D$ = 1,4848; $d^{20}$ = 0,9928;

IR: 1700, 815 $cm^{-1}$;

RMN (60 MHz): 0,86; 0,89; 0,91 (plusieurs singulets, 6H); 1,36 et 1,38 (2xd, J = 5,5 Hz, 3H); 2,75 et 3,02 (plusieurs m, 2H); 5,45 (m, 2H) δ ppm;

SM: M+ = 208 (1); m/e: 193(2), 175(1), 163(0,5), 152(7), 135(3), 123(100), 107(68), 95(21), 81(68), 67(17), 55(15), 41(35), 29(20).

**Exemple 2**

Composition parfumante pour savon

Une composition parfumante pour savon a été préparée en mélangeant les ingrédients suivants (parties en poids):

| | |
|---|---|
| Géranylacétone | 200 |
| Acétate de benzyle | 140 |
| Alcool phényléthylique | 100 |
| Acétoacétate d'éthyle | 100 |
| Diéthylacétal de l'ald. α-amylcinnamique | 80 |
| Triméthylcyclohexènecarbaldehyde à 10 %* | 60 |
| Verdox IFF[1] | 40 |
| Musc DTI[2) 3)] | 40 |
| Hédione[3) 4)] | 40 |
| Citronellol | 20 |
| Ald. α-hexylcinnamique | 10 |
| Epoxy-cétone de l'Ex. 1 | 170 |
| | 1000 |

* dans le phtalate diéthylique
1) acétate de 2-tert-butyl-1-cyclohexyle
2) 1,1 -diméthyl-4-acétyl-6-tert-butylindane
3) origine: Firmenich SA, Genève
4) dihydrojasmonate de méthyle

Le caractère fruité de la composition de base a été modifié par l'addition de l'époxy-cétone de l'invention qui sert à lui conférer une note agréable de type pomme. Si, à titre de comparaison, l'on remplace ladite époxy-cétone par de l'α-damascone, dans la même proportion, l'on obtient une nouvelle composition dont le caractère fruité est trop marqué, ce qui entraîne un manque d'harmonie olfactive.

**Exemple 3**

<u>Composition parfumante pour solution de mise en plis</u>

Une composition parfumante a été préparée en mélangeant les ingrédients suivants (parties en poids):

| | |
|---|---|
| Salicylate de benzyle | 150 |
| Acétate de bicyclopentadiényle | 80 |
| Géraniol | 80 |
| Essence de rose synth. | 60 |
| Acétate de 3,5,5-triméthylhexyle | 50 |
| Cyclopidène[1) 2)] à 10 %* | 50 |
| Essence de jasmin synth. | 40 |
| Lilial[3)] | 40 |
| Alcool cinnamique | 40 |
| Galbanum Résinoïde synth. | 30 |
| Lyral[4)] | 30 |
| Acétate de cyclohexyléthyle | 30 |
| α-Isométhylionone | 30 |
| Diméthylbenzylcarbinol | 30 |
| Mayol[5) 2)] | 20 |
| Essence de muguet synth. | 20 |
| Benzophénone | 20 |
| Citralva[6)] à 10 %* | 20 |
| Cyclogéraniate de méthyle à 10 %* | 20 |
| Galaxolide[4)] | 20 |
| Essence de patchouli à 10 %* | 20 |
| Coumarine à 10%* | 20 |
| Epoxy-cétone de l'Ex. 1 | 100 |
| | 1000 |

* dans le phtalate diéthylique
1) cyclopentylidène-acétate
2) origine: Firmenich SA
3) origine: L. Givaudan
4) origine: IFF
5) 4-isopropyl-cyclohexyl-méthanol
6) géranonitrile

L'addition de l'époxy-cétone confère à la composition de base brillance et arrondi à la note fruitée.

**Exemple 4**

Deux bases détergentes en poudre ont été préparées en mélangeant les ingrédients suivants (parties en poids):

| | Composition | Composition avec perborate de sodium |
|---|---|---|
| Alkyl-benzènesulphonate de sodium linéaire (longueur de la chaîne $C_{11-5}$) | 8,0 | 6,4 |
| Alcool de suif ethoxylé (14EO) | 2,9 | 2,3 |
| Savon sodique (longueur de chaîne $C_{12-16}$13-26 %; $C_{18-22}$74-87 %) | 3,5 | 2,8 |
| Triphosphate de sodium | 43,8 | 35,0 |
| Silicate de sodium | 7,5 | 6,0 |
| Silicate de magnésium | 1,9 | 1,5 |
| Carboxymethylcellulose | 1,2 | 1,0 |
| Sodium EDTA | 0,2 | 0,2 |
| Sulphate de sodium | 21,2 | 17,0 |
| Eau | 9,8 | 7,8 |
| Perborate de sodium | - | 20,0 |
| | 100,0 | 100,0 |

Lorsqu'on additione à un échantillon de l'une des poudres détergentes ci-dessus l'époxy-cétone de l'Exemple 1, à raison d'environ 1 % en poids, on lui confère une odeur fruitée puissante et élégante.

**Exemple 5**

Une base adoucissante pour textiles a été préparée en mélangeant les ingrédients suivants (parties en poids):

| Ingrédients | Parties | Origine |
|---|---|---|
| Praepagen WK | 10,0 | Hoechst |
| Emulsifiant O120 | 0,5 | Zschimmer & Schwarz |
| Polyglycol 400 | 2,0 | Hoechst |
| Eau distillée | 84,4 | |
| Colorant: | | |
| Brilliant Blau R 28032 sol. aqueuse à 0,5 % | 0,1 | Siegle |
| Chlorure de sodium sol. aqueuse à 10 % | 0,7 | |
| Poromycen F 10 | 0,1 | Kraft |
| Alcool isopropylique C+ | 2,0 | Shell |
| | 99,8 | |

L'addition à un échantillon de la base adoucissante indiquée ci-dessus du produit de l'Exemple 1, à raison de 1 %, lui confère une agréable odeur fruitée.

**Exemple 6**

L'epoxy-cétone de l'Exemple 1 a été employée pour le parfumage des produits indiqués ci-après. Les concentrations respectives utilisées du produit ainsi que la stabilité de l'odeur observée dans ces différents produits sont indiquées dans le tableau suivant:

| | concentration [%][1)] | stabilité[2)] |
|---|---|---|
| Eau de toilette | 5 | S/N |
| Crème de nuit | 0,4 | S/N |
| Schampoing | 0,5 | S/N |
| Désodorisant aérosol | 1,2 | S/N |
| Laque pour cheveux | 0,3 | S/N |
| Savon | 0,5 | S/N |
| Talc | 0,5 | I/N |
| Poudre à récurer chlorée | 0,2 | S/N |

1) en poids par rapport au poids total du produit terminé
2) mesurée après stockage du produit parfumé à 40° pendant 1 mois
S = stable; I = instable (se réfèrent à l'odeur)
N = normale (se réfère à la couleur)

**Revendications**

1. Composé de formule

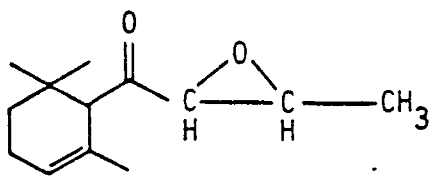

2. Ingrédient parfumant constitué par le composé selon la revendication 1.
3. Composition parfumante contenant à titre d'ingrédient actif le composé selon la revendication 1.
4. Article parfumé contenant à titre d'ingrédient parfumant actif le composé selon la revendication 1.
5. A titre d'article parfumé selon la revendication 4, une composition détergente liquide ou solide.
6. A titre d'article parfumé selon la revendication 4, une composition adoucissante pour textiles.
7. Procédé pour la préparation du composé selon la revendication 1, caractérisé en ce qu'on époxyde, au moyen d'un agent d'époxydation en milieu basique, l'α-damascone.
8. Procédé suivant la revendication 7, caractérisé en ce qu'on effectue l'époxydation à l'aide d'eau oxygénée ou d'un perborate de métal alcalin et que l'on opère en milieu aqueux ou aqueux-alcoolique.

## Patentansprüche

1. Verbindung der Formel

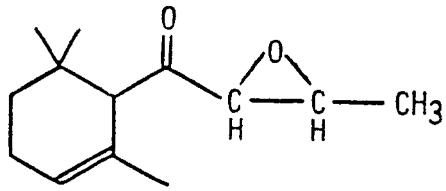

2. Parfümierender Bestandteil bestehend aus der Verbindung nach Anspruch 1.
3. Parfümkomposition, die als aktiven Bestandteil die Verbindung nach Anspruch 1 enthält.
4. Parfümierter Artikel, welcher als aktiven Bestandteil die Verbindung nach Anspruch 1 enthält.
5. Als parfümierten Artikel nach Anspruch 4, ein flüssiges oder festes Detergenz.
6. Als parfümierten Artikel nach Anspruch 4, einen Textilweichmacher.
7. Verfahren zur Herstellung der Verbindung nach Anspruch 1 dadurch gekennzeichnet, dass man mit Hilfe eines Epoxydationsmittels in basischem Milieu, alpha-Damascon epoxydiert.
8. Verfahren nach Anspruch 7, dadurch gekennzeichnet, dass man die Epoxydierung mit Hilfe von Wasserstoffperoxid oder einem Alkalimetallperborat in wässerigem oder wässerig/alkoholischem Milieu durchführt.

## Claims

1. Compound of formula

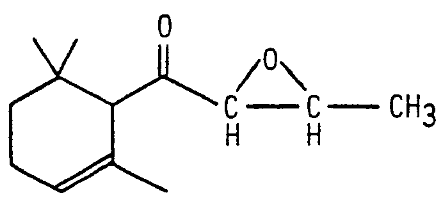

2. Perfuming ingredient consisting in the compound according to claim 1.
3. Perfuming composition containing as active ingredient the compound according to claim 1.
4. Perfumed article containing as active perfuming ingredient the compound according to claim 1.
5. As a perfumed article according to claim 4, a liquid or solid detergent composition.
6. As a perfumed article according to claim 4, a fabric softener composition.
7. Process for the preparation of a compound according to claim 1, characterized in that alpha-damascone is epoxydized by means of an epoxydizing agent in a basic medium.
8. Process according to claim 7, characterized in that the epoxydation is effected by means of hydrogen peroxide or of an alkali metal perborate and that it is carried out in an aqueous or aqueous-alcoholic medium.